Europäisches Patentamt

European Patent Office

Office européen des brevets

(19)

(11) Veröffentlichungsnummer: **0 004 845**

(12) **EUROPÄISCHE PATENTSCHRIFT**

(45) Veröffentlichungstag der Patentschrift: 03.02.82

(21) Anmeldenummer: 78101557.3

(22) Anmeldetag: 04.12.78

(51) Int. Cl.³: **A 61 B 10/00,** **G 10 K 11/18, A 61 N 1/04, A 61 H 23/00**

(54) **Behandlungskopf für elektromedizinische diagnostische oder therapeutische Behandlung von Körperteilen.**

(30) Priorität: 16.12.77 DE 7738446 U

(43) Veröffentlichungstag der Anmeldung:
31.10.79 Patentblatt 79/22

(45) Bekanntmachung des Hinweises auf die Patenterteilung:
03.02.82 Patentblatt 82/5

(84) Benannte Vertragsstaaten:
BE CH DE FR GB IT NL SE

(56) Entgegenhaltungen:
DE - B - 1 239 880
DE - C - 852 275
DE - C - 942 722
FR - A - 2 200 024
GB - A - 527 562
US - A - 3 533 397

(73) Patentinhaber: SIEMENS AKTIENGESELLSCHAFT
Berlin und München
Postfach 22 02 61
D-8000 München 22 (DE)

(72) Erfinder: Beyer, Johann
Schiessäckerstrasse 1
D-8521 Baiersdorf (DE)
Erfinder: Strauber, Friedrich
Im Herrenholz 8
D-8520 Buckenhof (DE)

## Behandlungskopf für elektromedizinische diagnostische oder therapeutische behandlung von körperteilen

Die Erfindung bezieht sich auf einen Behandlungskopf für elektromedizinische diagnostische oder therapeutische Behandlung von Körperteilen, insbesondere mit Ultraschall und/oder Reizstrom, mit einem Applikator mit erster Behandlungsfläche, dem ein Adapterstück mit von der ersten Behandlungsfläche unterschiedlicher Behandlungsfläche vorschaltbar ist, wobei das Adapterstück unmittelbar formschlüssig an die erste Behandlungsfläche anfügbar ist.

Behandlungsköpfe zur Verwendung an Körperteilen benötigen unterschiedliche Behandlungsflächen je nach Form der zu behandelnden Körperfläche. So wird man beispielsweise relativ flache Körperpartien, wie z.B. Bauch, Rücken oder Brustkorb, mit relativ großer Behandlungsfläche behandeln können; stärker gekrümmte Körperkonturen, wie beispielsweise Oberarmrundungen oder sonstige Gelenkformationen der Körperextremitäten, z.B. auch Nacken, Kniescheibe oder Fingergelenke, benötigen hingegen eine kleinere Behandlungsfläche. Bisher hat man sich zum Übergange von größerer auf kleinere Behandlungsfläche oder umgekehrt damit begnügt, für jede der benötigten Behandlungsflächen einen separaten Behandlungskopf bereitzustellen. Der Aufwand an technischem Zuberhör war dementsprechend groß.

Ein Behandlungskopf letzterer Art ist insbesondere aus der GB—A—527 562 vorbekannt. Bei diesem Behandlungskopf speziell für die Ultraschallbehandlung können wahlweise verschiedene Applikatoren mit unterschiedlich ausgeformten Behandlungsflächen formschlüssig auf dem eigentlichen Ultraschall-Wandler befestigt werden. Zur Befestigung der Applikatoren ist ein Anschraubmechanismus speziell als Bajonettverschluß ausgebildet.

Eine weitere Möglichkeit zur Umgehung obiger Nachteile besteht auch darin, daß einem einzigen Behandlungskopf mit erster Behandlungsfläche ein dazu passendes Adapterstück mit unterschiedlicher weiterer Behandlungsfläche vorschaltbar ist. Der Aufwand an technischem Zubehör wird dadurch zwar vermieden; problematisch ist dabei der Halterungsmechanismus zum formschlüssigen Verbinden der ersten Behandlungsfläche mit der Rückseite des Adapterstückes, welche beide zwecks störungsfreier Ultraschallübertragung plan aufeinander liegen müssen. Ein Anschraubmechanismus wäre zum Aufeinanderpressen beider Flächen mit erhöhtem Fertigungsaufwand verbunden und in der Handhabung auch recht umständlich.

Aufgabe vorliegender Erfindung ist es daher, einen Behandlungskopf dahingehend auszubilden, daß er ohne großen Aufwand mit einem einfachen Handgriff rasch auf beliebige unterschiedliche Behandlungsflächen bringbar ist.

Die Aufgabe ist bei einem Behandlungskopf der eingangs genannten Art erfindungsgemäß dadurch gelöst, daß die der ersten Bahandlungsfläche zugewandte Ankoppelungsfläche des Adapterstückes mit zweiter Behandlungsfläche eine wulstartig umlaufende Manschette trägt, deren über die erste Behandlungsfläche des Applikators schiebbarer Manschettenumfang mit einer Fingeranlage zum Anpressen oder Wiederabnehmen des Adapterstückes mit zweiter Behandlungsfläche versehen ist, und daß der die erste Behandlungsfläche aufweisende Applikator an seinem Umfang mit Gegenklemmitteln für die Manschette versehen ist.

Die Erfindung ermöglicht ohne allzu großen technischen Mehraufwand das Umstecken von erster auf zweite Behandlungsfläche oder umgekehrt rasch mit einfachem Handgriff. Zur guten Halterung und zum leichten Anstecken der Ansteckmanschette an das Adapterstück sollte in vorteilhafter Ausgestaltung der Erfindung die der ersten Behandlungsfläche zugewandte Ankoppelungsfläche an einer Schulter wenigstens eine vorzugsweise umlaufende Nut aufweisen, in die die Manschette mittels dazu passender erster Wulstlippe der Manschette einknöpfbar ist. Entsprechend sollte in weiterer vorteilhafter Ausgestaltung der Erfindung der Applikator mit erster Behandlungsfläche an seinem Umfang mit wenigstens einer Nut versehen sein, in die beim Anstecken des Adapterstückes mittels Manschette eine dazu passende zweite Wulstlippe der Manschette einschnappt. Vorteilhaft ist es auch, wenn die Fingeranlage zum Anpressen oder Wiederabnehmen des Adapterstückes als napfartig umlaufender Wulst der Ansteckmanschette ausgebildet ist. Insgesamt läßt sich dadurch die formschlüssige Verbindung von erster Behandlungsfläche mit der Ankopplungsfläche derart sicherstellen, daß zusätzliche Ultraschallübertragungsmittel nicht zwingend notwendig sind.

Weitere Vorteile und Einzelheiten der Erfindung ergeben sich aus der nachfolgenden Beschreibung eines Ausführungsbeispiels anhand der Zeichnung in Verbindung mit den weiteren Unteransprüchen. Es zeigen:

Fig. 1 einen Behandlungskopf speziell für Ultraschall und/oder Reizstrombehandlung mit angestecktem Adapterstück teilweise im Schnittbild,

Fig. 2 ein Adapterstück losgelöst von der Ansteckmanschette,

Fig. 3 ein Ansteckmanschette zum Anstecken eines Adapterstückes an den Behandlungskopf.

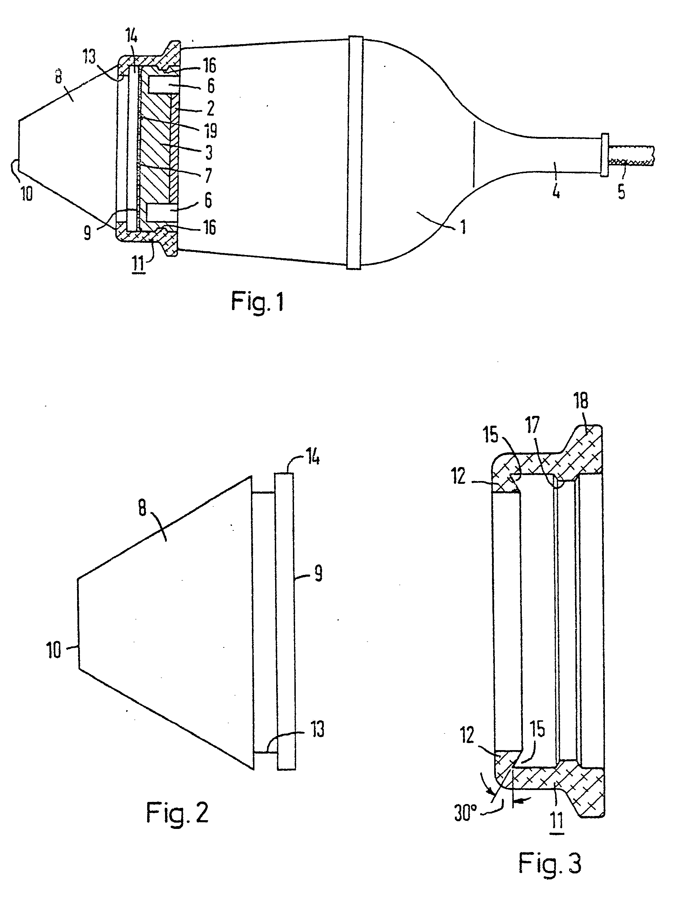

In der Fig. 1 ist mit 1 der Behandlungskopf bezeichnet. Der Behandlungskopf 1 trägt stirnseitig einen Ultraschallschwinger 2, der beispielsweise aus Bariumzirkonat oder Bariumtitanat besteht. Dem Ultraschallschwinger 2 ist an der Stirnfläche des Ultraschallkopfes 1 weiter ein erstes Anpassungsstück, vorzugsweise aus Aluminium, vorgeschaltet. Bei Behandlung mit Ultraschall hat dieses erste Anpassungsstück 3 eine Dicke, die ein Mehrfaches der halben Wellenlänge ($\lambda/2$) der Ultraschallwellen beträgt. Für Reizstrombehandlung anstelle von Ultraschallbestrahlung oder Reizstrombehandlung zusammen mit Ultraschallbestrahlung kann das Aluminiumanpassungsstück auch gleichzeitig Elektrode zur Übertragung der Reizströme sein. Zu diesem Zwecke weist also nicht nur der Schallschwinger einen elektrischen Anschluß für den (nicht dargestellten) Hochfrequenz-Erregungsoszillator auf. Auch das Adapterstück ist dann über eine entsprechende elektrische Leitung mit einem Reizstromgenerator verbunden. Sämtliche Anschlußleitungen sind über einen Kabelanschluß 4 des Ultraschallkopfes mit gemeinsamem Anschußkabel an die entsprechenden Versorgungseinrichtungenanschaltbar.

In der Fig. 1 weist das Anpassungsstück 3 des Behandlungskopfes 1 (das mit einem umlaufenden Lufthohlraum 6 zur Entkopplung der Seitenflächen versehen ist) eine erste Behandlungsfläche 7 auf. Diese erste Behandlungsfläche 7 des Behandlungskopfes 1 weist relativ große Abmessungen auf. Der Durchmesser liegt bei beispielsweise ca. 42 mm (bei vorausgesetzter Kreisfläche). Die sich hieraus ergebende Behandlungsfläche eignet sich besonders gut zur Adaption an relativ schwach gekrümmten Körperpartien.

Soll nun jedoch Adaption an stärker gekrümmten Körperpartien erfolgen, so wird dem Ultraschallkopf 1 an der ersten großflächigen Behandlungsfläche 7 ein Adapterstück 8 vorgeschaltet. Dieses Adapterstück 8, das vorzugsweise ebenfalls aus Aluminium besteht, hat in etwa die Form eines Kegelstumpfes. Dieser Kegelstumpf verjüngt sich demnach von einer größeren Ansetzfläche 9, die im aneinandergesteckten Zustand an der ersten Behandlungsfläche 7 des Behandlungskopfes anliegt, zu einer zweiten Behandlungsfläche 10 mit kleineren Abmessungen. Beträgt beispielsweise in Anpassung an die Behandlungsfläche 7 des Behandlungskopfes 1 die Ankoppelfläche 9 des Adapterstückes 8 im Durchmesser ebenfalls ca. 42 mm, so hat die zweite Behandlungsfläche (bei ebenfalls vorausgesetzter Kreisform) nur noch einen Durchmesser von etwa 12 mm. Es ist selbstverständlich, daß diese Abmessungen lediglich beispielshaften Charakter haben; sie können bei entsprechend geändertem Bedarf, beispielsweise bei Verwendung andersartiger Behandlungsköpfe oder auch anders gestalteter Adapterstücke, entsprechend anders dimensioniert sein.

Das Anstecken des Adapterstückes 8 an die erste Behandlungsfläche 7 des Behandlungskopfes 1 erfolgt mittels Ansteckmanschette 11. Die Ansteckmanschette 11 ist aus einem gummiartigen Material, insbesondere Silikonkautschuk oder Weichplastik, gefertigt. Zum Anstecken an die Ansteckfläche 9 des Adapterstückes 8 weist nun der Manschettenring 11 eine umlaufende Wulstlippe 12 auf, die in eine Umlaufnut 13 an einer Schulter 14 der Ansteckfläche 9 des Adapterstückes 8 paßt. In diese Nut 13 am Adapterstück 8 ist somit die Manschette 11 mittels Wulstlippe 12 einknöpfbar. In besonderer Ausgestaltung ist nun die Wulstlippe 12 auch noch mit einem Hinterschnitt 15 versehen, der in der dargestellten Weise ca. 30° beträgt, bei andersartiger Ausführung aber auch Winkel von beispielsweise 45° oder mehr einnehmen kann. Der Hinterschnitt gewähleistet die besonders gute Anpassung des Adapterstückes mit der Fläche 9 an die Behandlungsfläche 7 des Ultraschallkopfes auch bei relativ stark schwankenden Fertigungstoleranzen.

Zum Anstecken des Adapterstückes 8 mittels Ansteckmanschette 11 an die Behandlungsfläche 7 des Behandlungskopfes 1 weist nun auch die Behandlungsfläche 7 des Behandlungskopfes am Anpassungsstück 3 wenigstens eine Nut 16 auf, in die beim Anstecken der Manschette eine dazu passende Wulstlippe 17 der Manschette einschnappt. Die Nut 16 der Behandlungsfläche 7 und die dazu passende Wulstlippe 17 der Manschette 11 sind wiederum ringförmig umlaufend ausgebildet. Darüber hinaus ist die Ansteckmanschette aber auch noch am über die erste Behandlungsfläche 7 des Behandlungskopfes 1 schiebbaren Manschettenumfang mit einer Fingeranlage 18 zum Anpressen oder Wiederabnehmen des Adapterstückes 8 samt Manschette 11 versehen. Die Fingeranlage ist dabei gemäß Ausführung der Fig. 1 und 3 als napfrandartig umlaufender Wulst ausgebildet.

In den Fig. 2 und 3 sind Adapterstück 8 und Manschette 11 in Explosionsdarstellung als unmontierte Einzelteile nochmals nebeneinanderliegend dargestellt. Erwähnenswert ist noch, daß auch das Adapterstück 8 eine Länge aufweisen kann, die wiederum ein ganzzahliges Vielfaches der halben Wellenlänge der Ultraschallwellen bei Ultraschallabstrahlung beträgt. Länge und Fläche können aber auch so aufeinander abgestimmt sein, daß sich nach Vorschaltung des Adapterstückes an der zweiten Behandlungsfläche im wesentlichen dieselben Abstrahlintensitätsverhältnisse wie an der ersten Abstrahlfläche 7 des Kopfes 1 ergeben. Zur besseren Anpassung und Ultraschallübertragung sollte ferner die Ankopplung der Fläche 9 des Adapterstückes 8 an die Behandlungsfläche 7 des Ultraschallkopfes 1 nicht blank, sondern vielmehr mittels Ultraschall-Kontaktpaste oder Wasser erfolgen. In der Fig. 1 ist z.B. eine Kontaktpaste (Koppelgel) mit der Bezugsziffer 19 versehen.

## Patentansprüche

1. Behandlungskopf (1) für elektromedizinische diagnostische oder therapeutische Behandlung von Körperteilen, insbesondere mit Ultraschall und/oder Reizstrom, mit einem Applikator (3) mit erster Behandlungsfläche (7), dem ein Adapterstück (8) mit von der ersten Behandlungsfläche (7) unterschiedlicher Behandlungsfläche (10) vorschaltbar ist, wobei das Adapterstück (8) unmittelbar formschlüssig an die erste Behandlungsfläche (7) anfügbar ist, dadurch gekennzeichnet, daß die der ersten Behandlungsfläche (7) zugewandte Ankoppelungsfläche (9) des Adapterstückes (8) mit zweiter Behandlungsfläche (10) eine wulstartig umlaufende Manschette (11) trägt, deren über die erste Behandlungsfläche (7) des Applikators (3) schiebbarer Manschettenumfang mit einer Fingeranlage (18) zum Anpressen oder Wiederabnehmen des Adapterstückes (8) mit zweiter Behandlungsfläche (10) versehen ist, und daß der die erste Behandlungsfläche (7) aufweisende Applikator (3) an seinem Umfang mit Gegenklemmitteln (16) für die Manschette (1) versehen ist.

2. Behandlungskopf nach Anspruch 1, dadurch gekennzeichnet, daß die der ersten Behandlungsfläche (7) zugewandte Ankopplungsfläche (9) an einer Schulter (14) wenigstens eine vorzugsweise umlaufende Nut (13) aufweist, in die die Manschette (11) mittels erster dazu passender Wulstlippe (12) einknöpfbar ist.

3. Behandlungskopf nach Anspruch 1 oder 2, dadurch gekennzeichnet, daß der Applikator (3) mit erster Behandlungsfläche (7) an seinem Umfang mit wenigstens einer Nut (16) versehen ist, in die beim Anstecken des Adapterstückes (8) mittels Manschette (11) eine dazu passende zweite Wulstlippe (17) der Manschette (11) einschnappt.

4. Behandlungskopf nach Anspruch 3, dadurch gekennzeichnet, daß die Nut (16) an dem die erste Behandlungsfläche (7) aufweisenden Applikator (3) und die dazu passende Wulstlippe (17) an der Manschette (11) ringförmig umlaufend ausgebildet sind.

5. Behandlungskopf nach Anspruch 4, dadurch gekennzeichnet, daß die Fingeranlage (18) ein napfartig umlaufender Wulst der Manschette ist.

6. Behandlungskopf nach Anspruch 2, dadurch gekennzeichnet, daß die erste Wulstlippe (12) mit einem Hinterschnitt (15) versehen ist.

7. Behandlungskopf nach Anspruch 6, dadurch gekennzeichnet, daß der Hinterschnitt (15) im Bereich von ca. 30° bis 45° liegt.

8. Behandlungskopf nach einem der Ansprüche 1 bis 7, dadurch gekennzeichnet, daß die Manschette (11) aus einem gummiartigen Material, insbesondere aus Silikonkautschuk oder Weichplastik, besteht.

9. Behandlungskopf nach einem der Ansprüch 1 bis 8, dadurch gekennzeichnet, daß die erste Behandlungsfläche (7) des Behandlungskopfes (1) sowie das daran ansteck- bzw. anklemmbare Adapterstück (8) mit zweiter Behandlungsfläche (10) aus einem Leichtmetall, vorzugsweise aus Aluminium, gefertigt sind.

10. Behandlungskopf nach einem der Ansprüche 1 bis 9, dadurch gekennzeichnet, daß speziell für Ultra-schallbehandlung der erste Applikator (3) am Kopfteil (1) mit erster Behandlungsfläche (7) sowie das an die erste Behandlungsfläche (7) ansteckbare bzw. anklemmbare Adapterstück (8) mit zweiter Behandlungsfläche (10) jedes für sich Längen aufweisen, die ganzzahlige Vielfache der halben Wellenlänge ($\lambda/2$) der abzuschallenden Ultraschallwellen betragen.

11. Behandlungskopf nach einem der Ansprüche 1 bis 10, dadurch gekennzeichnet, daß speziell für Ultraschallbehandlung das Adapterstück (8) mit zweiter Behandlungsfläche (10) in Länge und Fläche so abgestimmt ist, daß sich beim Anklemmen bzw. Anstecken an die erste Behandlungsfläche (7) des Behandlungskopfes (1) an der zweiten Behandlungsfläche (10) des Adapterstückes (8) im wesentlichen dieselben Abstrahlintensitätsverhältnisse wie an der ersten Abstrahlfläche (7) des Kopfteils (1) ergeben.

## Claims

1. A treatment head (1) for the electromedical diagnostic or therapeutic treatment of parts of the body, in particular with ultrasonic vibration and/or a stimulating current, comprising an applicator (3) having a first treatment surface (7), to which can be connected an adaptor member (8) having a second treatment surface (10) which differs from the first treatment surface (7), wherein the adaptor member (8) can be directly attached to the first treatment surface (7) so as to interlock therewith by virtue of its shape, characterised in that the coupling surface (9), of the adaptor member (8) which faces the first treatment surface (7) carries a collar-like sleeve (11) the periphery of which can be slid over the first treatment surface (7) of the applicator (3) and is provided with a finger means (18) for use in pressing on or removing the adaptor member (8), and that the periphery of the applicator (3) is provided with counter-gripping means (16) for the sleeve (11).

2. A treatment as claimed in Claim 1, characterised in that at a shoulder (14), the coupling surface (9) has at least one groove (13) running round it, into which groove the sleeve (11) can be snapped by means of a first, corresponding bead-shaped lip (12).

3. A treatment head as claimed in Claim 1 or Claim 2, characterised in that, at its periphery, the applicator (3) is provided with at least one groove (16) into which a corresponding second bead-shaped lip (17) of the sleeve (11) snaps when the adaptor component (8) is fastened on by means of the sleeve (11).

4. A treatment as claimed in Claim 3, characterised in that the groove (16) in the applicator (3) and the corresponding bead-shaped lip (17) on the sleeve (11) are of annular shape.

5. A treatment head as claimed in claim 4, characterised in that the finger means (18) consists of a bowl-shaped bead running round the sleeve.

6. A treatment head as claimed in Claim 2, characterised in that the first bead-shaped lip (12) is provided with an undercut (15).

7. A treatment head as claimed in Claim 6, characterised in that the undercut (15) is in the range of from 30° to 45°.

8. A treatment head as claimed in one of Claims 1 to 7, characterised in that the sleeve (11) is made of a rubber-like material, in particular made of silicone rubber, or soft plastics material.

9. A treatment head as claimed in one of Claims 1 to 8, characterised in that the first treatment surface (7) of the treatment head (1) and the adaptor member (8) which can be fixed or clamped thereto, are made of a soft metal, preferably aluminium.

10. A treatment head as claimed in one of Claims 1 to 9, characterised in that, in particular for ultrasonic treatment, the first applicator (3) on the head portion (1) having the first treatment surface (7), and the adaptor member (8) which can be fixed or clamped onto the first treatment surface (7) and which itself is provided with the second treatment surface (10), each has a length which is a whole-numbered multiple of half the wavelength ($\lambda/2$) of the ultrasonic waves to be used.

11. A treatment head as claimed in one of Claims 1 to 10, characterised in that, in particular for ultrasonic treatment, the adaptor member (8) is adapted in respect of its length and area, to be such that when it is clamped or fixed onto the first treatment surface (7) of the treatment head (1), basically the same irradiation intensity conditions prevail at the second treatment surface (10) of the adaptor member (8) as at the first treatment surface (7) of the head portion (1).

## Revendications

1. Tête (1) permettant d'effectuer un traitement thérapeutique ou de diagnostic médical par voie électrique, notamment à l'aide d'ultrasons et/ou d'un courant d'excitation, comprenant un applicateur (3), qui a une première face (7) de traitement et en avant duquel peut être monté un adapteur (8) ayant une face (10) de traitement différente de la première face (7), l'adapteur (8) pouvant être réuni directement par complémentarité de forme à la première face (7), caractérisée en ce que la face (9) d'accouplement, se trouvant du côté de la première face (7) de traitement, de l'adapteur (8) ayant la seconde face (10) de traitement porte une garniture (11) sans fin, en forme de bourrelet, dont le pourtour, qui peut se déplacer sur la première face (7) de traitement de l'applicateur (3), est muni d'un appui (18) pour le doigt en vue de presser ou de relâcher l'adapteur (8) ayant la seconde face (10) de traitement et le pourtour de l'applicateur (3) ayant la première face (7) de traitement est muni de moyens (16) conjugués de blocage de la garniture (11).

2. Tête suivant le revendication 1, caractérisée en ce que la face (9) d'accouplement se trouvant du côté de la première face (7) de traitement présente sur un épaulement (14) au moins une gorge (13), de préférence sans fin, dans laquelle la garniture (11) peut être boutonnée à l'aide d'une première lèvre (12) en bourrelet s'y adjustant.

3. Tête suivant la revendication 1 ou 2, caractérisée en ce que le pourtour de l'applicateur (3) ayant la première face (7) de traitement est muni d'au moins une gorge (16), dans laquelle, lorsque l'adapteur (8) est enfiché à l'aide de la garniture (11), s'encliquète une seconde lèvre en bourrelet s'y adaptant de la garniture (11).

4. Tête suivant la revendication 3, caractérisée en ce que la gorge (16) sur l'applicateur ayant la première face (7) de traitement et la lèvre (17) en bourrelet s'y ajustant sont ménagées de manière annulaire et sans fin sur la garniture (11).

5. Tête suivant la revendication 4, caractérisée en ce que l'appui (18) pour le doigt est un bourrelet sans fin en forme de nez de la garniture.

6. Tête suivant la revendication 2, caractérisée en ce que la première lèvre (12) en bourrelet est munie d'une contre-dépouille (15).

7. Tête suivant la revendication 6, caractérisée en ce que la contre-dépouille (15) fait un angle de 30° à 45° environ.

8. Tête suivant l'une des revendications 1 à 7, caractérisée en ce que la garniture (11) est en une matière semblable à du caoutchouc, notamment en caoutchouc de silicone ou en matière plastique molle.

9. Tête suivant l'une revendications 1 à 8, caractérisée en ce que la première face (7) de traitment de la tête (1) ainsi que l'adapteur (8), qui peut y être enfiché ou y être bloqué et qui a la seconde face (2) de traitement, sont en un métal léger, de préférence en aluminium.

10. Tête suivant l'une des revendications 1 à 9, caractérisée en ce que spécialement pour le traitement par les ultrasons, le premier applicateur (3) sur la partie (1) de la tête ayant la première face (7) de traitement ainsi que l'adapteur (8), qui a la seconde face (10) de traitement et qui peut s'enficher ou se bloquer sur la première face (7) de traitement, ont chacun des longueurs qui correspondent à un

multiple entier de la demi-longueur d'onde ($\lambda/2$) des ondes ultrasonores qui se propagent.

11. Tête suivant l'une des revendications 1 à 10, caractérisée en ce que spécialement pour le traitement par les ultrasons, l'adapteur (8) ayant la seconde face (10) de traitement est accordé en longueur et en surface de manière à ce que, lorsqu'il est bloqué ou enfilé sur la première face (7) de traitement de la tête (1), il y a sur la seconde face (10) de traitement de l'adapteur (8) essentiellement le même rapport d'intensité de rayonnement que sur la première face (7) de traitement de la partie (1) de la tête.

Fig. 1

Fig. 2

Fig. 3